# EUROPEAN PATENT SPECIFICATION

(11) **EP 2 042 205 B1**
(45) Date of publication and mention of the grant of the patent: **21.09.2011**
(21) Application number: 08016963.4
(22) Date of filing: 26.09.2008
(51) Int. Cl.: A61M 5/315, A61M 5/31, A61M 5/32, A61M 39/20, A61D 7/00, A61D 1/02

(54) **Syringe with cap and cap removal device**
Spritze mit Kappe und Kappenentfernungshilfsmittel
Seringue avec capuchon et outil pour enlever le capuchon

(30) Priority: 27.09.2007 NZ 56203707
(43) Date of publication of application: 01.04.2009
(73) Proprietor: Merial Limited, Duluth, GA 30096 (US)
(72) Inventor: Smith, Robert, Balmain, NSW 2041 (AU); Holmes, Robert William Lachlan, Birkenhead Auckland (NZ)
(74) Representative: Harding, Charles Thomas

(56) References cited:
- US-A- 3 212 685
- US-A- 5 087 249
- US-A- 5 279 577
- US-A- 5 292 313
- US-B1- 6 283 946

## Description

### FIELD OF THE INVENTION

This invention relates to syringes, caps and cap removed devices therefor, particularly but not necessarily solely for use in veterinary applications.

### BACKGROUND OF THE INVENTION

Syringes for injecting substances are well known. In general they consist of a barrel with a tip for dispensing a substance, or a connector suitable for receiving a needle, located at one end, and a plunger which contacts and seals with the interior surface of the barrel at the other end. Finger grips extend radially from the surface of the barrel at the end of the barrel which receives the plunger. The plunger is actuated by the thumb pressing against the end of the plunger. When fully actuated the end of the plunger and the finger grips are adjacent one another.

To provide controlled operation of a syringe during the dispensing procedure the syringe must be kept stable. By "stable" is meant having minimal or no unwanted movement during the dispensing procedure.

Of particular interest are syringes used in the veterinary field for the intramammary application of treatments to prevent or control mastitis, for example in dairy cows. These syringes typically contain an antibiotic paste or gel. To administer this material the veterinarian or farmer typically removes the cap from the syringe to expose a narrow tip that can vary in length from approximately 5 mm to 15 mm. While holding the teat of the cow in one hand and the syringe in the other, the tip of the syringe is inserted into the teat opening. The plunger of the syringe is depressed with the thumb, finger or base of hand. When the contents of the syringe have been fully dispensed the tip is withdrawn and the syringe discarded.

Survey studies in New Zealand have shown that there are a number of methods employed by users to both remove the cap of the syringe and to hold the syringe and dispense the contents.

It was found that: 23.5% of users gripped the cap in their teeth in order to remove it; 14.6% of users pushed the cap off with their thumb in a sideways movement; and 61 % of users held the syringe in one hand while pulling the cap off with the other. All users expressed frustration with existing syringe designs because due to the nature of the environment in which they are applied the caps are very slippery to grip. This makes them very difficult to remove.

Users also expressed frustration with the difficulty with which syringes could be held during use. 85% of users held the syringe in what could be classified as the traditional manner with their fingers on the finger grips and thumb on the plunger. 10% of users held the syringe with their fingers on the finger grips and the base of the plunger in the palm of their hand. In the case of intramammary syringes where the cow may be moving around it is important that the syringe can be held firmly at all times. Another particular reason for this is that it is critical that no contamination or bacteria is introduced onto the tip of the syringe during the dispensing procedure. If the syringe is not able to be held firmly it may brush the outside of the udder or the cow's leg or other barrel part, thereby causing contamination of the syringe.

Prior art syringe arrangements do not allow for controlled operation of a syringe during the dispensing procedure, due to the relative positions of the finger grips and the end of the plunger when the syringe is fully actuated. As they are adjacent one another, the dispensing end of the barrel (which may or may not be fitted with a needle) is unsupported during the operation of the syringe, leaving the syringe unstable. If the syringe is unstable, it is difficult to obtain and maintain the correct injection site and this may cause discomfort to the recipient of the injection, together with the problems mentioned above.

Keeping a syringe stable also assists the user in injecting the correct amount of the substance.

In addition, prior art syringes are not ideal for dispensing substances such as viscous pastes and the like. This is because the thumb acting on the plunger has to provide sufficient force to move the plunger down towards the dispensing end of the barrel; an operation which can be inefficient and uncomfortable.

Various syringe- and syringe-cap designs as well as cap removal devices are disclosed in e.g. US 5 292 313, US 5 087 249, US 5 279 577, WO 03/051423 and US 2006/0260275. Ergonomic Syringe designs are disclosed in e.g. US 3 212 685 and US 6 283 946.

There is a need for a syringe that is more stable than prior art syringes during the injection procedure and which is efficient, comfortable and easy to use irrespective of the substance being administered.

Syringe caps which are currently in use are generally designed to be pulled or pushed or severed off the tip of the syringe prior to use. Accordingly, they can be difficult and tiresome to remove, particularly when using syringes repeatedly during farming operations. Usually they are pushed off with the thumb prior to use, which can cause damage to the syringe tip (bending or breakage) and can also present a contamination risk as the user's thumb or fingers may come into contact with the syringe tip during removal.

The discussion herein of the background to the invention is included to explain the context of the invention. This is not to be taken as an admission that any of the material referred to was published, known or part of the common general knowledge as of the priority date of any of the claims of the invention.

### SUMMARY OF THE INVENTION

The above mentioned problems are solved by the substance delivery system according to claim 1. It comprises a syringe, a cap and a cap removal device. The syringe comprises a body, comprising a conduit therethrough and two openings, a gripping section and an elongated plunger or piston, comprising an engagement section, an elongated section and a seal assembly. The syringe functions in the normal way known to one skilled in the art.

The syringe includes a gripping section which allows a user to grip the body with one or more digits of a user's hand. The gripping section may be positioned at any point along the length of the body. Some embodiments may be configured to maintain a predetermined separation distance between the gripping section and the rear end of the plunger or piston. Alternatively, some embodiments may be configured to maintain a separation distance between an end of the body and an end of the plunger or piston. Additionally, the body of the syringe may have a preselected overall length.

The plunger or piston is adapted to be reciprocated through a predefined stroke length to move the seal assembly through the conduit within the body. The seal assembly may thus be moved from a fully retracted position to a fully depressed position to drive a substance present in the body out an opening positioned in the dispensing end of the body. The plunger or piston may be moved by applying pressure to the engagement section disposed at a rear end of the elongated section. Pressure may be applied to the engagement section with any surface, including but not limited to a digit or heel of the user's hands.

In some embodiments, the plunger or piston has a length that is substantially 110% or greater than the preselected overall length of the body. Thus, the engagement section may extend beyond an end of the body a predetermined distance when the plunger or piston is in the fully depressed position.

In an embodiment, the plunger or piston has a length that is greater than about 120% of the preselected overall length of the body. The length of the plunger or piston may be greater than about 130%, 150%, 170%, 190%, 210%, 230%, 250%, 270% or 290% of the preselected overall length of the body.

In an embodiment, a portion of the plunger or piston proximate the engagement section is enlarged with respect to the rest of the plunger or piston for increased stability during use. For example, the plunger or piston may have a flared end or a tapered outer periphery such that the diameter of the plunger or piston increases along its length towards the engagement section.

In an embodiment, the engagement section has a larger diameter than the rest of the plunger or piston for ease of use. The engagement section may be slightly concaved to accommodate application of pressure by the user comfortably during use.

The substance delivery system according to the invention includes a cap, which is adapted to removably couple to the body to protect the opening when not in use. In some embodiments, a retaining assembly may be disposed on the cap and/or the body.

An embodiment may include a cap adapted to removably couple to the body having an opening to protect the opening when not in use. In some embodiments, a retaining assembly may be disposed on the cap and/or the body.

The cap includes a receiving portion which receives at least a portion of the body therein in an insertion direction. The receiving portion may have a retaining assembly adapted to removably grip the body when present in the receiving portion with a retaining force adapted to removably retain the cap to the body.

Further, a leveragable portion or remover allows the cap to be removed from the body. The leveragable portion has geometry according to claim 1.

The system according to the invention further includes a cap removal device adapted to remove the cap from the body. The cap removal device includes a support portion adapted to be supported and to support a cantilever portion. The cantilever portion is adapted to allow the cap to be removed from the body. The cantilever portion in combination with the support portion forms a first receiving recess. The cantilever portion has two prongs forming a second recess therebetween, such that when the remover of the cap is inserted in the first receiving recess and the receiving portion of the cap is inserted in the second recess, the cap can be removed from the syringe body.

The support portion is furthermore supported by means of a band member adapted to be attached to a wrist or belt of the user.

### BRIEF DESCRIPTION OF THE DRAWINGS

Throughout the body of this description reference will be made to the accompanying drawings in which:
Figure 1 is an isometric view of the embodiment of the syringe,
Figure 2 is a perspective view of a syringe body in accordance with an embodiment of the present invention,
Figure 3 is a top view of the embodiment of the syringe as shown in Figure 1,
Figure 4 is a perspective view of a syringe plunger or piston in accordance with an embodiment of the present invention,
Figure 5 is a front view of a syringe and plunger or piston in a fully depressed position in accordance with an embodiment of the present invention,
Figure 6 is a cross-sectional view of the embodiment of the syringe as shown in Figure 5, along the line X-X',
Figure 7 is a side view of the embodiment of the syringe as shown in Figure 5,
Figure 8 is a front view of a syringe (including a cap) and plunger or piston in a fully retracted position in accordance with an embodiment of the present invention,
Figure 9 is a cross-sectional view of the embodiment of the syringe as shown in Figure 8, along the line Y-Y',
Figure 10 is a side view of the embodiment of the syringe as shown in Figure 1,
Figure 11 is a perspective view of the embodiment of the syringe shown in Figure 1,
Figure 12 is a perspective view of an unassembled cap and syringe including the body and plunger or piston,
Figure 13 is a perspective view of the cap, body and plunger or piston shown in Figure 13 as assembled,
Figures 14a-e are a series of views of a cap including a cross-sectional view of the embodiment of the cap as shown in Figure 14a, along the line Z-Z',
Figure 15 is a side view of a syringe with a flared cap,
Figure 16 is a side view of a syringe with a cap not according to the invention having a frusto-conical shaped leveragable portion,
Figure 17 is a side view of a syringe with a cap having a leveragable portion with a disc-shaped end,
Figure 18 is a perspective view showing the cap being removed from the syringe by a user,
Figure 19 is a perspective view of a cap removal device according to the invention adapted to be worn around a wrist of a user, and
Figure 20 is a perspective view of a further embodiment of a cap removal device according to the invention, adapted to be worn on a belt of a user.

### DETAILED DESCRIPTION

Referring to the drawings, like numerals designate corresponding parts where possible throughout the several figures.

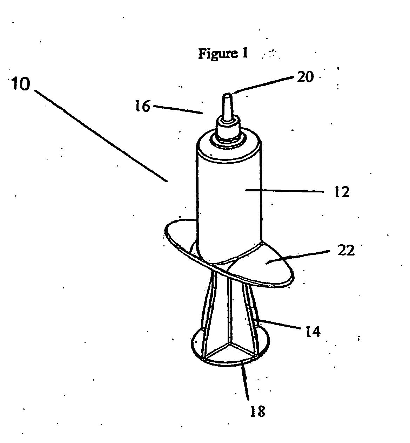

An embodiment of a syringe is depicted in Figure 1. Syringe 10 includes body 12 and plunger or piston 14. Plunger or piston 14 is positioned within body 12 such that dispensing end 16 of the body and rear end 18 of the plunger or piston are located opposite ends of the syringe.

Body 12 includes an opening 20 at dispensing end 16 of syringe 10. In some examples, multiple openings may be disposed at the end of the syringe. Alternately, some examples may include one or more openings proximate dispensing end 16. Openings may be utilized for dispensing substances including but not limited to medicines. In some embodiments, fluids, including but not limited to viscous fluids such as pastes, gels may be dispensed using the syringe. In some examples, dispensing end 16 and/or opening 20 may include a needle. In some examples, the dispensing end of the syringe may include a needle, such as a hypodermic needle, a nozzle, and/or tubing to help direct the flow into and out of the body.

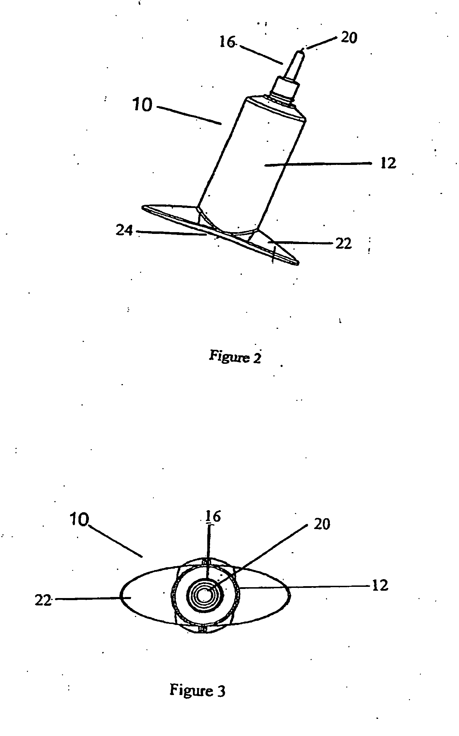

As shown in Figure 2, body 12 includes gripping section 22 to allow a user to grip the body 12. For example, user may use one or more digits of user's hand. Gripping section 22 may be disposed on body 12 such that the fingers can support syringe 10. Gripping section 22 may assist in obtaining the correct administration site and keeping the syringe substantially stable during operation. In the embodiment shown in Figure 2, gripping section 22 includes two protrusions. In an embodiment, gripping section 22 may include a pair of opposing projections which extend at an angle from the surface of the body 12 as shown in Figure 3. In some embodiments, the gripping section may extend substantially radially outward from the body of the syringe. For example, a gripping section may include a protrusion including, but not limited to a flange, rim, collar, and/or ring. In some embodiments, the gripping section may be ergonomically designed to accommodate a user's digits and/or hands comfortably. For example, a gripping section may have indentations or curves designed to conform with the shape of a hand or fingers. Use of fingers and/or a hand may support and guide the syringe more effectively during operation due to the design of the syringe. Accordingly, substances may be administered more accurately. In some embodiments, having the ability to activate the syringe by the heel of the hand, may make the operation of the syringe more comfortable to the user.

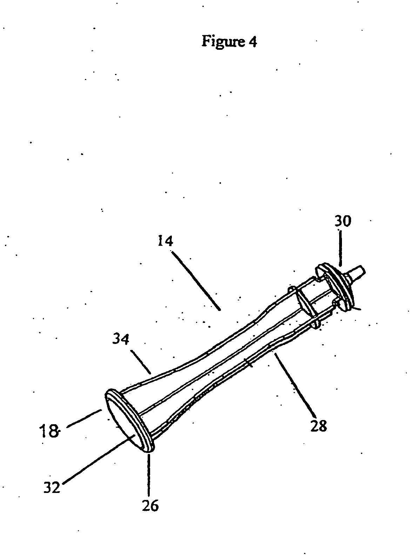

Body 12 is adapted to receive plunger or piston 14 at end 24 of the body as shown in Figure 2. Plunger or piston 14 depicted in Figure 4 includes engagement section 26, elongated section 28, and seal assembly 30. As shown in Figures 3-10, the diameter of plunger or piston 14 may vary along its length. Thus, the plunger or piston may have one or more sections which flare outward. Alternately, the plunger or piston may have sections in which the cross-sectional diameter increases or decreases.

As shown in Figure 4, the plunger or piston 14 has an engaging member 32 disposed at rear end 18 for engaging with a digit or heel of a user's hand as the user reciprocates the plunger or piston 14. In some embodiments, the engaging member 32 may be enlarged so that it can be comfortably engaged with the heel of the user's hand during use. For example, engaging member 32 may have a larger diameter than the rest of plunger or piston 14. Engaging member 32 may have geometry conducive to applying pressure. In some embodiments, engaging member 32 may be slightly concaved to accommodate the user's thumb, fingers and/or hand comfortably during use.

It will be obvious to those skilled in the art that the engaging member does not have to be operated by the heel of a hand, as the thumb or another digit may be used instead. In some embodiments, using the heel of the hand to depress the engaging member into the body towards the dispensing end may provide increased pressure, and is thus may make it easier and more comfortable to use. In the case of farming operations, ease of use may be valuable where syringes are used repeatedly for an extended period of time.

As shown in Figure 4, plunger or piston (14) may include flared section 34 proximate the engaging member 32. Flared section 34 may provide increased stability during use. In some embodiments, the diameter of the plunger or piston may vary along its length. Alternatively, the plunger or piston may have a flared end. In some embodiments, plunger or piston 14 may have a tapered outer periphery such that the diameter of the plunger or piston increases along its length towards the engaging member.

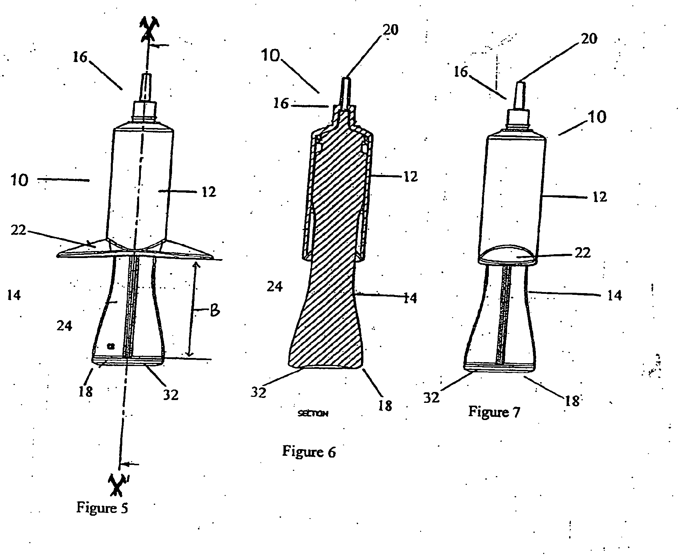

Figures 5 to 7 depict plunger or piston 14 positioned within body 12 in the fully depressed position. As shown in Figures 5 to 7, engaging member 32 and gripping section 22 may be spaced from one another by a predetermined separation distance ("B") (see Figure 5). As shown in the cross-sectional depiction of Figure 6, when fully depressed the configuration of the plunger or piston may maintain the predetermined separation distance ("B") between engaging member 32 and gripping section 22.

Plunger or piston 14 may be positioned within the conduit of the syringe body. In some examples, the profile of the elongated member may substantially conform to the profile of the conduit. In alternate examples, the profile of the elongated member may conform to portions of the conduit as depicted in Figure 6. The conduit communicates with opening 20.

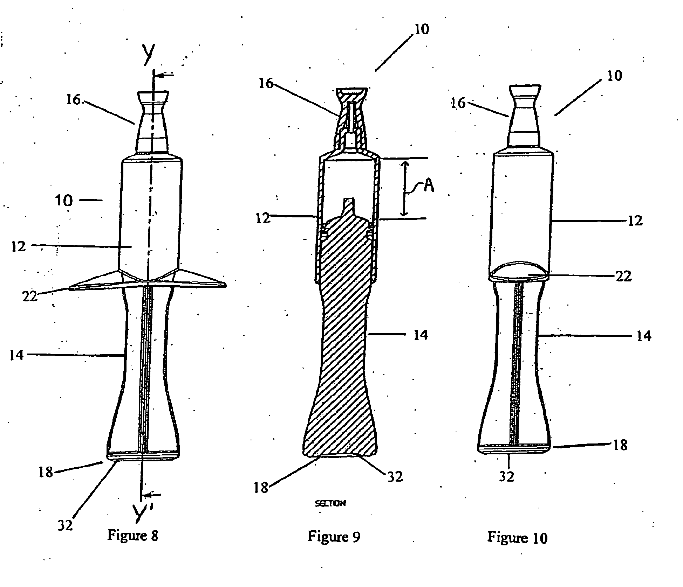

Figures 8-10 depict the plunger or piston positioned within the body.

As shown in Figure 6 and Figure 9, plunger or piston 14 is adapted to be reciprocated through a predefined stroke length to move seal assembly 30 through the internal volume of body 12 from a fully retracted position (See Figure 9) to a fully depressed position (See Figure 6) to drive a substance when present in body 12 through opening 20. The predefined stroke length ("A") (See Figure 9) may be the distance plunger or piston 14 is moved from the fully retracted position as shown in Figure 9 to the fully depressed position as shown in Figure 6.

A stroke length may be defined as the distance indicated as "A" in Figure 9. In some examples, the stroke length may be predefined. In some examples, a separation distance ("B") (as shown in Figure 5) may be equal to the value of at least 50% of a predefined stroke length ("A") (as shown in Figure 9).

In an example, the separation distance ("B") may be even greater. In some examples, a separation distance may be greater than about 60%, 80%, 100%, 150%, 200% or 250% of the predefined stroke length. For example, the separation distance ("B") may be at least 2 cm. In alternate examples, the separation distance ("B") may be greater than that. For example, a separation distance ("B") may be greater than about 3 cm, 4 cm, 5 cm, 6 cm, 7 cm, 8 cm, 9 cm or 10 cm, depending on the desired overall dimensions of the syringe. Alternately, in some examples the stroke length may vary.

In an embodiment, the separation distance ("B") (See Figure 5) may be achieved by providing plunger or piston 14 which has a length that is at least 120% of the predefined stroke length ("A") (See Figure 9). In other examples, the length of the plunger or piston may be even greater, such as for example, greater than about 150%, 200%, 250%, 300%, 350% or 400% of the predefined stroke length. Accordingly, when the plunger or piston 14 is in the fully depressed position, the engaging member 32 is disposed outward from the body 12 to result in the engaging member 32 being disposed away from gripping section 22 by the predetermined separation distance ("B") as shown in Figure 5.

Described in another way, the plunger or piston may have a length that is substantially 110% or greater than the overall length of the body (which is preselected depending on the desired overall dimensions of the syringe), such that the engaging member is disposed outward from an end of the body a predetermined distance when the plunger or piston is disposed in the fully depressed position. In some embodiments, the plunger or piston has a length that is greater than about 120% of the preselected overall length of the body. For example, the elongated member may have a length that is greater than about 130%, 150%, 170%, 190%, 210%, 230%, 250%, 270% or 290% of the preselected overall length of the body.

The syringe (10) includes a cap. Caps according to the invention are designed to be easily removed prior to use such that removal of the cap does not cause damage and/or contamination to the dispensing end of the syringe. Caps may be removable, reusable and/or configured for single use.

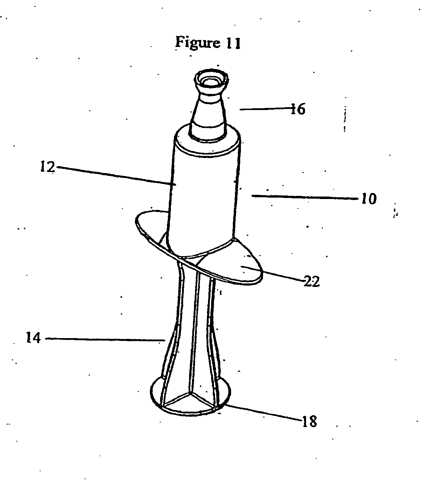

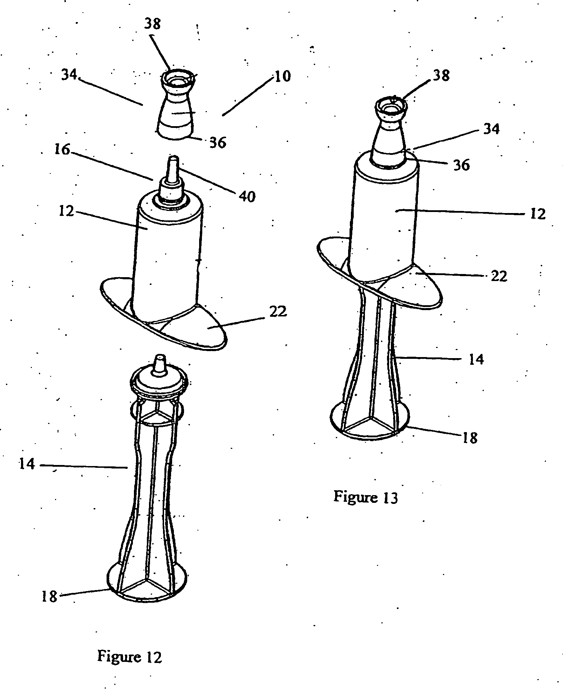

Figures 8 to 17 show various examples and embodiments of caps 34 which may be used to protect the tips of syringes. Figure 12 shows the syringe comprising a plunger or piston 14, body 12 and cap 34 in an unassembled form. As shown in Figure 12, each cap has proximal end 36 which will be positioned closest to body 12 and a distal end 38 positioned near the tip 40 when the cap is in place. In some examples, cap 34 may be configured to conform to profile of tip 40. Figure 13 shows the syringe and cap as assembled and ready to use.

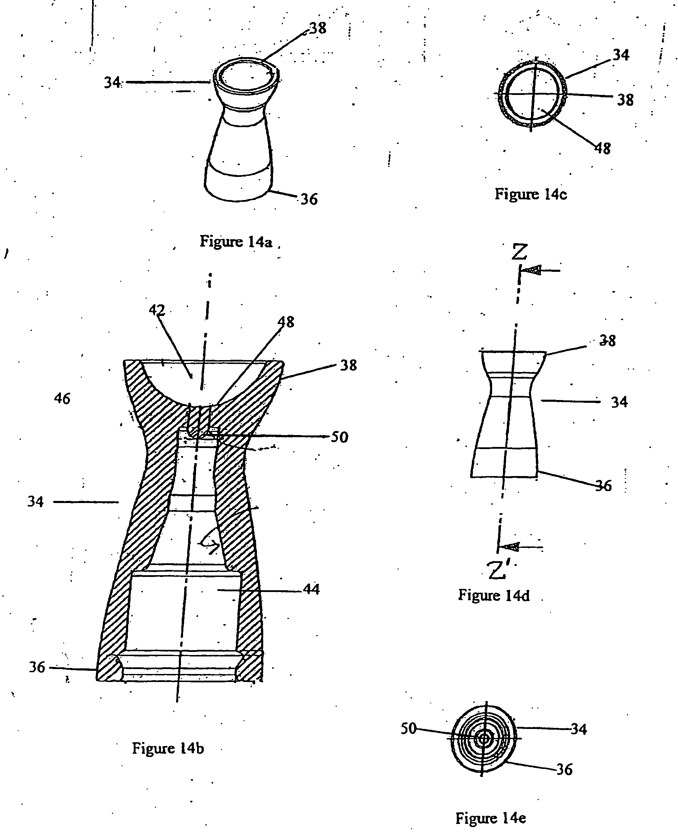

Figure 14 depicts various views of cap 34. Figure 14a depicts a perspective view of cap 34. As shown in Figure 14a, cap 34 may be contoured. Cap 34 has proximal end 36 closest to body 12 and includes a substantially hollow and substantially outwardly contoured, elongate structure. The elongate structure may define a length and have closed surface 42 at distal end 38. Figure 14b shows a cross-sectional view of the cap depicted in Figure 14d along line Z-Z'. Receiving portion 44 is coupled to top portion 46. As shown in Figures 14b and 14e, each cap has a hollow receiving portion at the proximal end 36 for receiving the syringe tip, and a top portion at the distal end 38 which provides the means for enabling removal of the cap from the syringe. The top portion may include a stop to prevent the tip of the substance delivery device from protruding therefrom. The stop can include any planar member with or without a recess or recesses. The stop may be curved therein. In one example the stop may include a circumferential rim. Top portion 46 may include an end stop. End stops may include, but are not limited to various surface profiles such as flush, recessed, planar, projections and/or curved surfaces. In addition, end stops may include outer stopping surface 48 and/or inner stopping surface 50. For example, as shown in Figure 14b inner stopping surface 50 is oriented substantially at right angles to the length of the substance delivery device and cap. In some examples, inner stopping surface 50 may engage tip 40 of syringe as shown in Figure 9.

A receiving portion of a cap may have an internal shape that allows the cap to couple to the syringe. For example, as shown in Figure 14b receiving portion 44 of cap 34 includes an inner portion shaped in a complementary shape to the substance delivery device outlet. In some examples, the cap may be removably held by a retaining assembly (not shown) in place over the tip of the substance delivery device.

In some examples, a portion of the cap may removably grip a portion of the syringe. Alternately, a portion of the ssyringe may be configured to grip a portion of the cap. The receiving portion of the cap may include a retaining assembly adapted to removably grip the syringe when present in the receiving portion with a retaining force adapted to removably retain the cap to the syringe. The retaining assembly may include any suitable configuration that allows for easy removal of the cap in use, yet sufficient cap retention when not in use. For example, retaining assembly may include, but are not limited to fastening mechanisms, friction fits, interference fits, press fits, click in place type mechanisms, and/or any other mechanism currently known or yet to be discovered in the art.

The cap protects the syringe and the contents of the syringe from contamination. Further, the cap may protect the user of the syringe. In use, when fitted to a syringe, the proximal end of the cap may abut the body of the syringe.

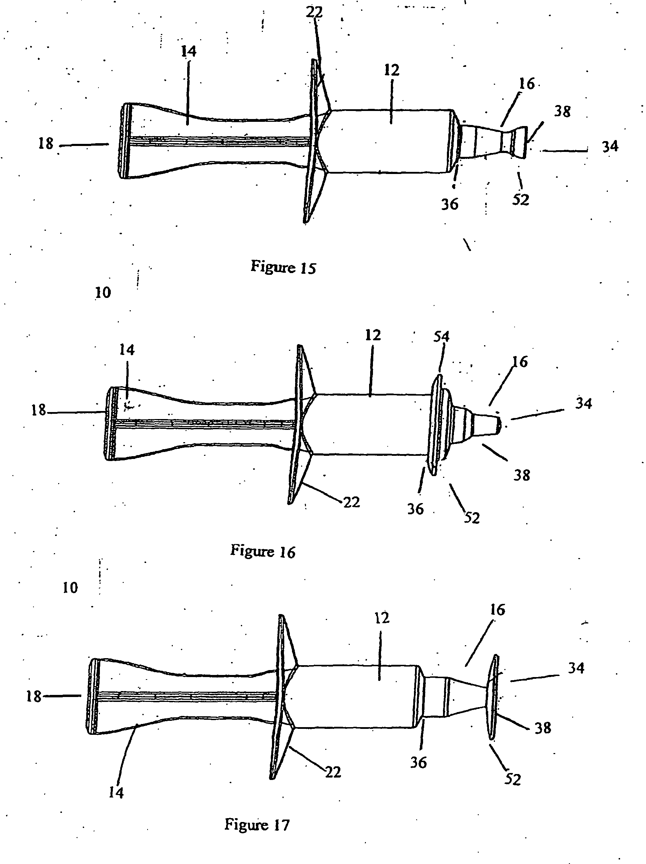

Figures 15-17 show various examples and embodiments of caps 34 having different profiles having leveragable portion or remover 52. In some examples, leveragable portion 52 may be ergonomically shaped to enable a user to easily grip the cap and remove it. In addition, a leveragable portion or remover 52 according to the invention includes, at least one flange, rim, projection projection, ring, and/or collar.

Figure 15 depicts a contoured cap having an ergonomic shape. In addition, the cap substantially conforms to the shape of the syringe tip. In Figure 16, cap 34 which is not according to the invention outwardly and inwardly substantially mirrors the shape of the syringe tip. Figure 16 includes cap 34 having flared rim 54 at the proximal end 36. In Figure 17, cap 34 has leveragable portion 52 at the distal end 38 which is disc-shaped to enable a user to easily grip the cap so it can be removed from the syringe.

In use, if the syringe is fitted with a cap as herein described, then the cap is first removed from the syringe. The syringe is then gripped using the fingers and the heel of the hand. The heel on the palm of a hand activates the engaging member by pushing it towards the dispensing end of the syringe while the user simultaneously grips the flanges of the gripping section with their fingers.

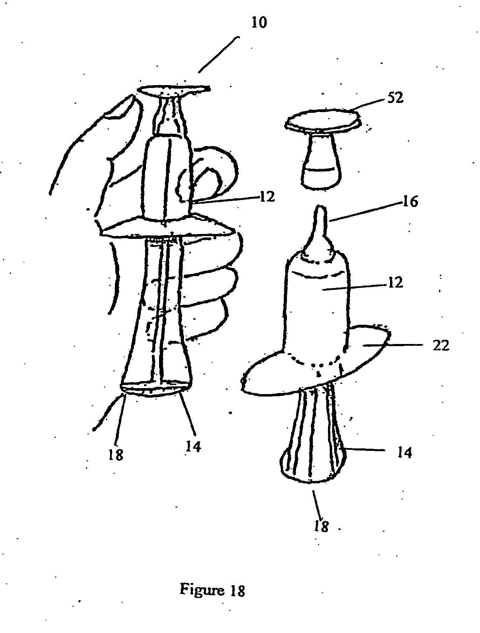

In order to remove any of the caps described herein, a user may simply grip the substance delivery device body and use their thumb to remove the cap, in a simple 'flicking' motion, as shown in Figure 18.

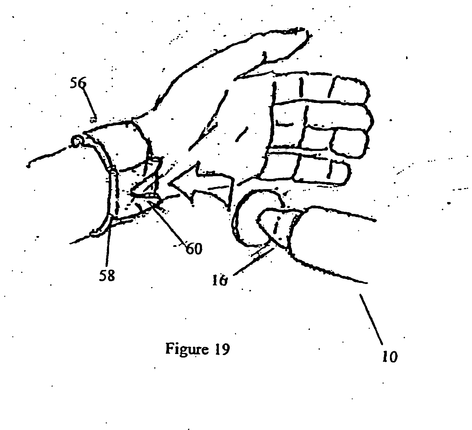

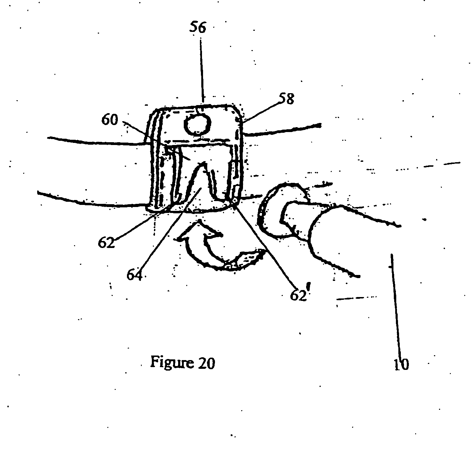

Figures 19 and 20 depict embodiments of cap removal devices 56 according to the invention. The cap removal devices comprise support portion 58 and a cantilever portion 60 having a space or a first receiving recess therebetween. Support portion 58 is supported by means of a band member which can be worn around a user's wrist (as shown in Figure 19), or a band member which can be slidably attached to a user's belt (as shown in Figure 20). Not according to the invention, support portion 58 could be adapted to be attached to another object or an upstand or the like. For example, the support portion may include, but is not limited to a hook shaped member and/or a member that is coupled using a fastener to a surface such as, a fence post, wall, framing, and/or any other convenient edge.

Cantilever portion 60 is shaped in such a way that the cap according to the invention may be inserted therein whilst still attached to the syringe, to enable removal of the cap. As shown in Figure 20, cantilever portion 60 includes two spaced apart prongs 62, 62' with recess 64 therebetween. The prongs are spaced from support portion 58 to define a first receiving recess to allow the leveragable portion or remover of cap 34 to be inserted behind the prongs and abut support portion 58 such that at least part of the body of the cap is accommodated in recess 64 between prongs 62, 62'. Once the leveragable portion or remover of the cap is in this position the user only then needs to pull in the direction parallel to the length of the syringe, to remove the cap.

As shown in Figures 19 and 20, the method of removing a cap from a syringe, utilizing a cap removal device as described herein, includes supporting the support portion 58 of the cap removal device by attaching it to a wrist or belt inserting the leveragable portion or remover of the cap into the first receiving recess of the cap removal device such that the hollow receiving portion of the cap is accommodated by the second recess, pulling the syringe/cap assembly until the cap is removed from the syringe.

The syringe may provide stability during the injection procedure and allow for controlled administration. When additional stability is provided, the injection procedure may be more effective and there may be less risk of contamination. In addition, activating the plunger or piston by engaging the heel of the hand with the engaging member may create more pressure and result in easier and less tiresome operation of the syringe. This may be particularly useful when syringes are being used repeatedly for extended time periods. According to the invention the syringe is be designed to be comfortable and easy to use.

In addition, a cap and cap removal device is provided which requires less effort to remove the cap from the syringe, and which results in less hand fatigue during repeated operations. Both the design of the cap and/or the use of the cap removal device provides for easier removal of the cap in cases where the cap is slippery. Also, there is less risk that the user's hands or fingers will contact the dispensing tip of the syringe body and cause contamination or damage (such as bending or breakage) to the dispensing tip.

An improved substance delivery system for use in veterinary field, for example for the intramammary application of treatments, is thus provided.

## Claims

1. A substance delivery system comprising:
a syringe (10) comprising:
a body (12) comprising:
a conduit therethrough;
an opening (20) at a first end of the body in communication with the conduit;
an aperture disposed at a second end (24) of the body in communication with the conduit; and
a gripping section (22);
an elongated plunger or piston (14) comprising:
an engagement section (26);
an elongated section (28); and
a seal assembly (30);
and a cap (34) comprising:
a proximal end (36) and a distal end (38), the distal end (38) providing a remover (52) comprising at least one protrusion, projection, flange, rim, ring and/or collar configured to allow removal of the cap (34);
a receiving portion (44) with
an opening disposed in the proximal end (36) of the receiving portion (44) for attachment to the first end of the syringe (10);
and a cap removal device (56) comprising:
a cantilever portion (60) configured to allow the cap (34) to be removed from the syringe (10);
a support portion (58) configured to provide support to the cantilever portion; and a receiving recess for receiving the remover (52) of the cap (34), wherein the receiving recess is formed at a juncture of the cantilever portion (60) and
the support portion (58),wherein the cantilever portion (60) further comprises two prongs (62, 62') forming a second recess (64) there between configured to engage the receiving portion of the cap (34), wherein the two prongs (62, 62') are arranged such that the cap (34) is engaged with them in a motion substantially perpendicular to the longitudinal axis of the syringe (10) such that the user needs to pull in the direction parallel to the length of the syringe (10) to remove the cap (34), and wherein the support portion (58) is supported by means of a band member adapted to be attached to a wrist or belt of the user.

2. The substance delivery system of claim 1 wherein the elongated plunger or piston (14) of the syringe (10) has a length that is 110% or greater than a pre-selected overall length of the body (12) such that the engagement section (26) is disposed outward from the second end (24) of the body (12) a predetermined distance when the elongated plunger or piston (14) is disposed in the fully depressed position and/or wherein the elongated plunger or piston (14) has a length that is greater than 120% of the pre-selected overall length of the body (12).

3. The substance delivery system of claim 1 or 2, wherein the elongated plunger or piston (14) of the syringe (10) is configured to maintain a predetermined separation distance between the gripping section (22) and the first end of the elongated member (14) and/or wherein the elongated plunger or piston (14) is configured to be reciprocated through a predefined stroke length to move the seal assembly (30) through the conduit of the body (12) from a fully retracted position to a fully depressed position to drive a substance when present in the body (12) out of the body (12) through the opening (20).

4. The substance delivery system of claim 1 or claim 2 or claim 3 wherein the engagement section (26) of the plunger or piston (14) is configured to engage a digit or heel of the user's hand as the user reciprocates the elongated plunger or piston (14).

5. The substance delivery system according to any one of claims 1 to 4, wherein the gripping section (22) is disposed at the second end (24) of the body (12).

6. The substance delivery system according to any one of claims 1 to 5, wherein a portion (34) of the elongated plunger or piston (14) proximate the engagement section (26) is enlarged with respect to the rest of the elongated plunger or piston (14) for increased stability during use and/or wherein the elongated plunger or piston (14) comprises a tapered outer periphery such that the diameter of the elongated plunger or piston (14) increases along the length of the elongated plunger or piston (14) towards the engagement section (26) and/or wherein the elongated plunger or piston (14) comprises a flared end.

7. The substance delivery system according to any one of claims 1 to 6, wherein a diameter of the engagement section (26) of the plunger or piston (14) has a larger diameter than the rest of the elongated plunger or piston (14) and/or wherein at least a portion of the engagement section (26) is concave.

8. The substance delivery system according to any one of claims 1 to 7, wherein the engagement section (26) of the plunger or piston (14) is configured to conform to a user's digit.

9. The substance delivery system according to any one of claims 1 to 8, wherein the syringe contains a substance comprising a viscous fluid and/or a gel and/or a viscous paste.

## Patentansprüche

1. Zuführungssystem für eine Substanz, das aufweist:
eine Spritze (10), die aufweist:
einen Körper (12), der aufweist:
einen Kanal durch ihn hindurch;
eine Öffnung (20) an einem ersten Ende des Körpers in Verbindung mit dem Kanal;
eine Mündung, die an einem zweiten Ende (24) des Körpers in Verbindung mit dem Kanal angeordnet ist; und
einen Greifabschnitt (22);
einen länglichen Stempel oder Kolben (14),
der aufweist:
einen Eingreifabschnitt (26) ;
einen länglichen Abschnitt (28); und
eine Dichtungsanordnung (30);
und eine Kappe (34), die aufweist:
ein proximales Ende (36) und ein distales Ende (38), wobei das distale Ende (38) einen Entferner (52) bereitstellt, der wenigstens einen Überstand, Vorsprung, Flansch, Rand, Ring und/oder eine Manschette aufweist, der/die das Entfernen der Kappe (34) zulässt;
einen Aufnahmeabschnitt (44) mit einer Öffnung, die für die Befestigung an dem ersten Ende der Spritze (10) in dem proximalen Ende (36) des Aufnahmeabschnitts (44) angeordnet ist;
und eine Kappenentfernungseinrichtung (56), die aufweist:
einen Auskragungsabschnitt (60), der aufgebaut ist, um zu ermöglichen, dass die Kappe (34) von der Spritze (10) entfernt winden Halteabschnitt (58), der aufgebaut ist, um einen Halt für den Auskragungsabschnitt bereitzustellen; und eine Aufnahmeaussparung zum Aufnehmen des Entferners (52) der Kappe (34), wobei die Aufnahmeaussparung an einem Übergang des Auskragungsabschnitts (60) und des Halteabschnitts (58) ausgebildet ist, wobei der Auskragungsabschnitt (60) ferner zwei Zacken (62, 62') aufweist, die eine zweite Aussparung (64) dazwischen bilden, die aufgebaut ist, um in den Aufnahmeabschnitt der Kappe (34) einzugreifen, wobei die zwei Zacken (62, 62') derart angeordnet sind, dass die Kappe (34) in einer Bewegung im Wesentlichen senkrecht zu der Längsachse der Spritze (10) mit ihnen in Eingriff gebracht wird, so dass der Benutzer in die Richtung parallel zu der Länge der Spritze (10) ziehen muss, um die Kappe (34) zu entfernen, und wobei der Halteabschnitt (58) mittels eines Bandelements gehalten wird, das geeignet ist, an einem Handgelenk oder einem Gürtel des Benutzers angebracht zu werden.

2. Zuführungssystem für eine Substanz nach Anspruch 1, wobei der längliche Stempel oder Kolben (14) der Spritze (10) eine Länge hat, die 110% oder größer als eine vorausgewählte Gesamtlänge des Körpers (12) ist, so dass der Eingreifabschnitt (26) in einem vorgegebenen Abstand auswärts von dem zweiten Ende (24) des Körpers (12) angeordnet ist, wenn der längliche Stempel oder Kolben (14) in der ganz niedergedrückten Position ist und/oder wobei der längliche Stempel oder Kolben (14) eine Länge hat, die größer als 120% der vorgeschriebenen Gesamtlänge des Körpers (12) ist.

3. Zuführungssystem für eine Substanz nach Anspruch 1 oder 2, wobei der längliche Stempel oder Kolben (14) der Spritze (10) aufgebaut ist, um einen vorgegebenen Trennungsabstand zwischen dem Greifabschnitt (22) und dem ersten Ende des länglichen Elements (14) aufrecht zu erhalten, und/oder wobei der längliche Stempel oder Kolben (14) aufgebaut ist, um durch eine vordefinierte Hublänge hin und her bewegt zu werden, um die Dichtungsanordnung (30) durch den Kanal des Körpers (12) aus einer ganz zurückgezogenen Position in eine ganz niedergedrückte Position zu bewegen, um eine Substanz, wenn diese sich in dem Körper (12) befindet, aus dem Körper (12) heraus durch die Öffnung (20) zu treiben.

4. Zuführungssystem für eine Substanz nach Anspruch 1 oder Anspruch 2 oder Anspruch 3, wobei der Eingreifabschnitt (26) des Stempels oder Kolbens (14) aufgebaut ist, um in einen Finger oder Ballen der Hand des Benutzers einzugreifen, während der Benutzer den länglichen Stempel oder Kolben (14) bewegt.

5. Zuführungssystem für eine Substanz nach einem der Ansprüche 1 bis 4, wobei der Greifabschnitt (22) an dem zweiten Ende (24) des Körpers (12) angeordnet ist.

6. Zuführungssystem für eine Substanz nach einem der Ansprüche 1 bis 5, wobei ein Abschnitt (34) des länglichen Stempels oder Kolbens (14) in der Nähe des Eingreifabschnitts (26) in Bezug auf den Rest des länglichen Stempels oder Kolbens (14) für eine erhöhte Stabilität während der Verwendung vergrößert ist und/oder wobei der längliche Stempel oder Kolben (14) einen abgeschrägten Außenumfang aufweist, so dass der Durchmesser des länglichen Stempels oder Kolbens (14) entlang der Länge des länglichen Stempels oder Kolbens (14) in Richtung des Eingreifabschnitts (26) zunimmt, und/oder wobei der längliche Stempel oder Kolben (14) ein aufgeweitetes Ende aufweist.

7. Zuführungssystem für eine Substanz nach einem der Ansprüche 1 bis 6, wobei ein Durchmesser des Eingreifabschnitts (26) des Stempels oder Kolbens (14) einen größeren Durchmesser als der Rest des länglichen Stempels oder Kolbens (14) hat, und/oder wobei wenigstens ein Abschnitt des Eingreifabschnitts (26) konkav ist.

8. Zuführungssystem für eine Substanz nach einem der Ansprüche 1 bis 7, wobei der Eingreifabschnitt (26) des Stempels oder Kolbens (14) aufgebaut ist, um sich dem Finger eines Benutzers anzupassen.

9. Zuführungssystem für eine Substanz nach einem der Ansprüche 1 bis 8, wobei die Spritze eine Substanz enthält, die eine viskose Flüssigkeit und/oder ein Gel und/oder eine viskose Paste aufweist.

## Revendications

1. Système de distribution de substance comprenant :
une seringue (10) comprenant :
un corps (12) comprenant :
un conduit à travers ce dernier ;
une ouverture (20) au niveau d'une première extrémité du corps en communication avec le conduit ;
une ouverture disposée au niveau d'une deuxième extrémité (24) du corps en communication avec le conduit ; et
une section de préhension (22) ;
un piston plongeur ou piston allongé (14) comprenant :
une section de mise en prise (26) ;
une section allongée (28) ; et
un ensemble de joint d'étanchéité (30) ;
et un capuchon (34) comprenant :
une extrémité proximale (36) et une extrémité distale (38), l'extrémité distale (38) fournissant un dispositif de retrait (52) comprenant au moins une protubérance, une saillie, un rebord, une collerette, une bague et/ou un collier configurés pour permettre le retrait du capuchon (34) ;
une partie de réception (44) avec une ouverture disposée dans l'extrémité distale (36) de la partie de réception (44) pour la fixation à la première extrémité de la seringue (10) ;
et un dispositif de retrait de capuchon (56) comprenant :
une partie en porte-à-faux (60) configurée pour permettre au capuchon (34) d'être retiré de la seringue (10) ;
une partie de support (58) configurée pour fournir le support à la partie en porte-à-faux; et un évidement de réception pour recevoir le dispositif de retrait (52) du capuchon (34), dans lequel l'évidement de réception est formé au niveau d'une jonction de la partie en porte-à-faux (60) et de la partie de support (58), dans lequel la partie en porte-à-faux (60) comprend en outre deux dents (62, 62') formant un deuxième évidement (64) entre elles, configurées pour mettre en prise la partie de réception du capuchon (34), dans lequel les deux dents (62, 62') sont agencées de sorte que le capuchon (34) est mis en prise avec elles dans un mouvement sensiblement perpendiculaire à l'axe longitudinal de la seringue (10) de sorte que l'utilisateur doit tirer dans la direction parallèle à la longueur de la seringue (10) pour retirer le capuchon (34), et dans lequel la partie de support (58) est supportée au moyen d'un élément formant bande adapté pour être fixé à un poignet ou à une ceinture de l'utilisateur.

2. Système de distribution de substance selon la revendication 1, dans lequel le piston plongeur ou piston allongé (14) de la seringue (10) a une longueur qui représente 110% ou plus d'une longueur globale présélectionnée du corps (12) de sorte que la section de mise en prise (26) est disposée vers l'extérieur à partir de la deuxième extrémité (24) du corps (12) à une distance prédéterminée lorsque le piston plongeur ou piston allongé (14) est disposé dans une position complètement enfoncée et/ou dans lequel le piston plongeur ou piston allongé (14) a une longueur qui est supérieure à 120% de la longueur globale présélectionnée du corps (12).

3. Système de distribution de substance selon la revendication 1 ou 2, dans lequel le piston plongeur ou piston allongé (14) de la seringue (10) est configuré pour maintenir une distance de séparation prédéterminée entre la section de préhension (22) et la première extrémité de l'élément allongé (14) et/ou dans lequel le piston plongeur ou piston allongé (14) est configurée pour effectuer un mouvement de va-et-vient sur une longueur de course prédéterminée afin de déplacer l'ensemble formant joint d'étanchéité (30) dans le conduit du corps (12) d'une position complètement rétractée à une position complètement enfoncée pour entraîner une substance, lorsqu'elle est présente dans le corps (12) à l'extérieur du corps (12) par une ouverture (20).

4. Système de distribution de substance selon la revendication 1 ou la revendication 2 ou la revendication 3, dans lequel la section de mise en prise (26) du piston plongeur ou piston (14) est configurée pour mettre en prise un doigt ou talon de la main de l'utilisateur, lorsque l'utilisateur fait effectuer un mouvement de va-et-vient au piston plongeur ou piston allongé (14).

5. Système de distribution de substance selon l'une quelconque des revendications 1 à 4, dans lequel la section de préhension (22) est disposée au niveau de la deuxième extrémité (24) du corps (12).

6. Système de distribution de substance selon l'une quelconque des revendications 1 à 5, dans lequel une partie (34) du piston plongeur ou piston allongé (14) à proximité de la section de mise en prise (26) est élargie par rapport au reste du piston plongeur ou piston allongé (14) pour une stabilité accrue pendant l'utilisation et/ou dans lequel le piston plongeur ou piston allongé (14) comprend une périphérie externe progressivement rétrécie de sorte que le diamètre du piston plongeur ou piston allongé (14) augmente le long de la longueur du piston plongeur ou piston allongé (14) vers la section de mise en prise (26) et/ou dans lequel le piston plongeur ou piston allongé (14) comprend une extrémité évasée.

7. Système de distribution de substance selon l'une quelconque des revendications 1 à 6, dans lequel un diamètre de la section de mise en prise (26) du piston plongeur ou piston (14) a un plus grand diamètre que le reste du piston plongeur ou piston allongé (14) et/ou dans lequel au moins une partie de la section de mise en prise (26) est concave.

8. Système de distribution de substance selon l'une quelconque des revendications 1 à 7, dans lequel la section de mise en prise (26) du piston plongeur ou piston (14) est configurée pour se conformer au doigt d'un utilisateur.

9. Système de distribution de substance selon l'une quelconque des revendications 1 à 8, dans lequel la seringue contient une substance comprenant un fluide visqueux et/ou un gel et/ou une pâte visqueuse.
